# EUROPEAN PATENT SPECIFICATION

(11) **EP 4 059 416 B1**
(45) Date of publication and mention of the grant of the patent: **27.08.2025**
(21) Application number: 21163378.9
(22) Date of filing: 18.03.2021
(51) Int. Cl.: A61B 5/0536, A61B 5/085, A61B 5/00

(54) **REGIONAL STRAIN**
REGIONALER STAMM
SOUCHE RÉGIONALE

(43) Date of publication of application: 21.09.2022
(73) Proprietor: SenTec AG, 4106 Therwil (CH)
(72) Inventor: Krukewitt, Lisa, 18057 Rostock (DE)
(74) Representative: Hepp Wenger Ryffel AG

(56) References cited:
- US-A1- 2019 038 173
- US-A1- 2019 246 949
- US-A1- 2020 324 067

## Description

The invention relates to a device and a method for determination of a measure for the homogeneity of the lung based on electrical impedance tomography (EIT) data as well as to a computer program product.

Electrical impedance tomography (EIT) is a non-invasive imaging technique based on the application of current and measurement of voltage through electrodes attached to the body of a patient. EIT provides images of the distribution of conductivity, admitivity, impedivity, or resistivity, or changes thereof. In the following the measured values are called "impedance values" and said distribution is also referred to collectively as "electrical properties". The image is called the EIT image. The image or sequences of images show differences in the electrical properties of various body tissues, bones, skin, body fluids and organs, particularly the lungs, which are useful for monitoring the patient's condition.

A typical EIT arrangement is shown in WO 2015/048917 A1. A set of electrodes is arranged on a belt, such that they are placed at a certain distance from each other around the chest of a patient, in electrical contact with the skin. An electrical current or voltage input signal is applied alternatingly between different or all possible pairs of electrodes. While the input signal is applied to one of the pairs of electrodes, the currents or voltages between the remaining electrodes may be measured. The measured electrical voltages of the body section may be reconstructed into electrical properties or changes of electrical properties by a reconstruction algorithm for use by a data processor to obtain a representation of the distribution impedance values over a cross-section of the patient around which the electrode belt ring is placed. The electrical properties are displayed on a screen.

In the EIT process a number of impedance measurement values are recorded at for example 16 or 32 electrodes. From these impedance measurement values, the EIT image reconstruction algorithm may then produce two dimensional images comprising pixels representing properties of the lungs. The image may comprise 32 x 32 pixels which represent locations within and outside the lungs.

Information on electrical properties obtained by EIT can be projected into a cross-sectional image derived from an anatomical model in an anatomical context, showing for example contours representing the outer boundaries of the modelled organs within the electrode plane.

Contours of functional structures can be used to (automatically) cluster the pixels within such structures to form Regions Of Interest (ROI) that match with functionally meaningful anatomical structures such as the lungs. Signals of pixels falling within such ROIs can be identified and analysed separately for each one of the ROIs by automatic signal processing means and algorithms.

Up to 100 or even more tomographic EIT images per second may be reconstructed. These high time-resolution images reflect the regional electrical properties within the lung tissue, which are influenced by the respiratory and cardiac cycles. In contrast to CT scans EIT-images do not display morphological but functional information showing regional tidal ventilation, local lung recruitment, expiratory time constants or the distribution of lung perfusion. EIT also measures end expiratory lung impedance (EELI), a parameter which correlates with functional residual capacity (FRC) at the global level and/or with the end expiratory lung volume (EELV) on a regional or pixel level.

The capability of EIT provides information about ventilation at the level of individual pixels in the EIT image. However, currently no reliable conversion from impedance values to volume measurements exists, and therefore a direct calculation of volumes and volume changes from EIT data is not possible. DE102017007224 discloses to determine properties and property changes of at least two regional areas of the lung on the basis of EIT data which represent regional changes in lung compliance or elasticity. The method does not provide a criterion at the level of the entire lung.

EP3725222A1 relates to a system for real-time determination of local stress on a lung during artificial respiration. A specific electrical impedance value at a specific point in time is assigned to a specific EIT pixel. A local tidal volume value z is determined as a difference between the end-inspiratory electrical impedance (ZINSP) and the end-expiratory electrical impedance (ZEXSP) of the specific EIT pixel. A preliminary strain value is determined by dividing the local tidal volume by the end-inspiratory electrical impedance. Preliminary strain values may be normalized to a reference strain value, for example at a predetermined PEEP value. Changes of the condition of the lung may be monitored for a specific patient, but a criterion of status of the lung as such may not be derived.

US 2019246949A1 discloses a system and method for determining a potential lung recruitment value for a patient that includes during an applied first positive end expiratory pressure (PEEP) to the lungs of the patient, measuring a first end expiratory lung impedance (EELZ) of the lungs at the first PEEP; during an applied second PEEP, measuring a second EELZ at the second PEEP, determining a change in EELZ between the first PEEP and the second PEEP, determining a first chord-compliance of the lungs from pixels of a first electrical impedance tomography (EIT) image of the patient at the first PEEP, determining a second chord-compliance from a second EIT image of the patient at the second PEEP, determining a first index representing the change in EELZ, determining a second index representing a change in compliance, and based on the first index and the second index, determining a potential lung recruitment value for the patient.

US2020324067A1 discloses a system for real-time determination of a local strain of a lung during artificial ventilation. The system comprises a device for electrical impedance tomography (EIT), which device is configured to capture an electrical impedance distribution along at least one two-dimensional section through a human thorax, and further comprises a device for assigning the captured electrical impedance distribution, which device is configured to divide the captured electrical impedance distribution at different times during the artificial ventilation into a multiplicity of EIT pixels and to assign a specific value of the electrical impedance at a specific time to a specific EIT pixel.

US 2019038173 discloses a device which determines difference parameters, such as end-expiratory impedance values, on the basis of electrical impedance tomography (EIT) data of regions of the lungs of a living being. The EIT data are obtained by an electrical impedance tomography apparatus. A quantitative evaluation of changes in regions of the lungs in terms of hyperdistension or collapsing is provided based on the determined difference parameters.

It is an object of the present invention to overcome the drawbacks of the prior art and in particular to provide a device and a method which allow to provide meaningful data concerning the lung in a simple and reliable way.

According to the invention these and other objects are solved with a device and a method for determining a measure for the homogeneity of the lung based on electrical impedance tomography (EIT) data according to the independent claims.

The ratio of change in lung volume dV during ventilation to the resting lung volume V₀ or end-expiratory lung volume EELV is referred to as lung strain (Gattinoni et al. 2012, Stress and strain within the lung, Curr Opin Crit Care 18(1): 42-7).

The device of the present invention is based on the finding that the determination, monitoring and analysis of strain on a pixel level by EIT can provide meaningful information even if no specific values of volume can be determined with EIT.

Lung strain resulting from increased respiratory drive during mechanical ventilation may inflict high energy loads on lung tissue thereby increasing the risk for patient self-inflicted lung injury (Brochard et al. 2017, Mechanical ventilation to minimize progression of lung injury in acute respiratory failure, Am J Respir Crit Care Med 195(4): 438-42).

A global strain can be derived from the ratio of tidal volume VT divided by functional residual capacity FRC.

During mechanical ventilation, low total lung strain reduces the risk of ventilator induced lung injury (Protti et al. 2012, Lung stress and strain during mechanical ventilation: Any safe threshold?, Am J Respir Crit Care Med 185: 115). It is, however, largely unclear what the healthy boundaries of such a ratio dV/V₀ are and when strain becomes unphysiologically high (Gattinoni et al. 2012). In mechanically ventilated pigs, Protti et al. found no lung damage of strains below 1.5 and acute respiratory failure for strain values above 2.5, but a grey zone remains between these values (Protti et al. 2012; Gattinoni et al. 2012). However, Gattinoni points out that such high lung strain does not typically appear in mechanically ventilated patients and points to lung inhomogeneity as a possible primary cause of lung injury in mechanically ventilated patients (Gattinoni et al. 2012). So far, the common definition of strain as dV/V₀ at the level of the entire lung means that such inhomogeneities often go unnoticed as this would require regional lung strain measurements.

Therefore, when looking at strain as a cause of lung injury, especially in spontaneously breathing patients, the focus needs to be on the determination of regional strain and on identifying strain inhomogeneities. A homogeneous distribution of regional lung strain would mean that the ratio dV/V₀ of change in lung volume dV to the resting lung volume V₀ is approximately constant throughout the lung. Thus, dV needs to increase linearly with V₀ meaning that regions with high lung volume show larger ventilation.

US 20140316266 discloses to determine local lung strain, lung volume and changes in lung volume using ultrasound imaging.

The device according to the present invention comprises a data input unit.

The data input unit is designed for receiving EIT data obtained by means of an electrical impedance tomography apparatus comprising electrodes to be positioned on a patient.

The EIT data may be provided to the data input unit directly from the electro-impedance tomography apparatus or indirectly via data lines, signal lines or network connections.

The device may be associated with or comprise an electro-impedance tomography apparatus.

The data input unit is configured to receive and provide EIT data from at least one region of at least one lung of a living being over an observation period. The observation period typically lasts over a number of respiratory cycles. Preferably, EIT data are provided for a cross-section of at least one entire lung.

A calculation and control unit is connected to the data input unit. The calculation and control unit is configured to determine impedance values over the observation period for each pixel of the at least one region.

The calculation unit may assign EIT data measured in at least one observation plane to a region of interest such as the right and/or the left lung.

The impedance values are typically stored as a data set in a data memory or data storage area assigned to the calculation and control unit and are kept available for further data processing in the data memory or data storage area.

The calculation and control unit is further configured to determine an impedance amplitude value for each pixel of at least one lung.

The amplitude is indicative of a difference between a maximal impedance value and a minimal impedance value within a respiratory cycle. The amplitude may be defined based on absolute maxima and minima within a cycle, but also on averages or on the basis of successive maxima and minima. The impedance amplitude value may be determined by a mean impedance difference over the observation period or by the maximal difference occurring over the observation period.

The calculation and control unit is further configured to determine an end-expiratory impedance value for each pixel of at least one lung. The end-expiratory impedance values may be calculated as the average minimum of the impedance values over the observation period for each pixel.

The calculation and control unit is further configured to determine data on the basis of the impedance amplitude values and the associated end-expiratory impedance values individually for each pixel.

The data may be used for a mapping of the impedance amplitude values and/or the end-expiratory impedance values to each pixel.

The data may provide a representation of a local distribution of measured values.

The data may be used to provide a graphical representation showing the relation between the change in impedance and the end-expiratory lung impedance for each pixel. For an unaffected lung the impedance amplitude values and the end-expiratory impedance values are assumed to be correlated to a certain extent.

Based on the determined data, the calculation and control unit may provide at least one number or at least a range of numbers which are characteristic for assignment relationship between the impedance amplitude values and the associated end-expiratory impedance values for each pixel.

The calculation and control unit is further configured to generate a control signal on basis of the data. The control signal may be used for forwarding, storing and/or displaying the data or information derived from the data.

The data may be analysed by a skilled person, who may draw conclusions from the pattern of the data. Data may also be used for automatic control of medical devices.

In particular the calculation and control unit is further configured to determine if the data fulfil a predetermined criterion. The data may be e.g. compared with a predetermined criterion, such as a predetermined data pattern, a predetermined threshold number or a predetermined range. An inhomogeneity indicator may be generated if the criterion is not fulfilled.

The criterion may be predetermined on the basis of a control group of patients. The control group may consist of persons having unaffected lungs.

Impedance amplitude values and associated end-expiratory impedance values may be measured or determined for the control group.

The criterion may be derived from a local distribution of impedance amplitude values, associated end-expiratory impedance values and/or of data deduced from impedance amplitude values and associated end-expiratory impedance values over pixels of the lung.

The criterion may be derived from a distribution of impedance amplitude values over associated end-expiratory impedance values.

The comparison may provide at least one difference between data obtained for a patient and the criterion and/or a ratio between data obtained for a patient and the criterion.

In EIT, determining absolute values of impedance and from this, lung volumes is difficult, especially without calibration. Therefore, although distributions and changes of volume can be quantified, intra-patient comparability remains difficult. Although the changes of volume and the resting lung volume relate to the impedance amplitude values and the end-expiratory lung impedance respectively, these measurements cannot be converted to volume measurements. Especially values for end-expiratory impedance values may vary greatly in EIT recordings of even the same patient, which may cause vastly different strain values if calculated as a simple ratio of the impedance amplitude values and the end-expiratory lung impedance.

Therefore, absolute values of the ratio of the impedance amplitude values and the end-expiratory lung impedance may vary between measurements of patients with similar lung strain or even between successive measurements of the same patient.

Thus, instead of focusing on absolute strain values, the data derived from associated impedance amplitude values and end-expiratory impedance values and in particular from a local distribution of impedance amplitude values and end-expiratory impedance values within the lung and/or of a statistical distribution of values derived from impedance amplitude values and end-expiratory impedance values can be used as a measure of the inhomogeneity of the lung.

This makes the method less susceptible to distortion by single outliers.

In healthy lungs, strain is low and assumed to be approximately constant throughout the lung with high changes in lung volume occurring in areas of high resting lung volume.

To identify regions with higher strain than in the surrounding tissue, no absolute values of the change in lung volume or the resting lung volume are needed according to the present invention.

Rather, an analysis can be based on a pixel-wise comparison of parameters that can be measured by EIT.

The calculation and control unit is configured to perform a function fit of the impedance amplitude values in dependency of the end-expiratory impedance values for at least one lung and to obtain a fitted function, in particular to obtain parameters characterizing the fitted function.

As regions with high lung volume show larger ventilation, and larger impedance variations, for example a linear relation between the impedance amplitude values and the end-expiratory impedance values may be assumed.

For example a linear regression may be performed, for example to obtain a linear function ΔZ_{ideal} = α EELI + β for at least one lung.

The fitted values represent a constant strain in each lung.

Even though impedance changes measured by EIT depend on a variety of patient-specific factors, such as thorax shape, making a calculation of tidal volume from EIT at least difficult if not almost impossible without calibration, it has been found that the impedance amplitude value can be assumed to increase linearly with the change in lung volume.

EIT provides a distribution of impedance changes proportional to regional tidal volumes. In the same manner, it may be assumed that end-expiratory lung impedance increases linearly with end-expiratory lung volume. It may be assumed that the distribution of end-expiratory lung impedance is proportional to the regional resting lung volume.

From the assumption of a constant ratio of change in lung volume and resting lung volume, a linear relationship between the impedance amplitude values and the end-expiratory impedance values can be assumed.

Depending on the reconstruction of the EIT image areas with an end-expiratory impedance close to zero do not show ventilation in healthy individuals since such high conductivities are not typical for healthy lung tissue. Therefore the offset β can be assumed zero and the ratio between the impedance amplitude values and the end-expiratory impedance values can be assumed to be a constant.

To identify regions with higher strain than in surrounding lung tissue absolute values for the regional distribution of changes in lung volume and resting lung volume are not needed. Rather, only information about the deviation of these parameters from a predefined, e.g. linear relationship at the regional level is needed.

As derived above, the distribution of tidal volume and lung volume within the lung relates to values that can be measured by EIT: tidal variation of impedance and end-expiratory impedance of each pixel. Therefore, EIT could provide real-time measurements of regional distribution of lung strain.

The calculation and control unit may further be configured to determine a distribution of impedance amplitude values with respect to the fitted values and to produce a control signal on basis of the data. For example, impedance amplitude values and fitted values may be assigned to a respective end-expiratory impedance value for each pixel. Alternatively or additionally, impedance amplitude values and fitted values may be assigned to the respective pixels. The distribution of impedance amplitude values may be compared with the distribution of fitted values.

The distribution of the impedance amplitude values may provide a measure for the status of the lung. When the distribution is sufficiently close to the fitted values which represent an ideal status, it can be assumed that the lung has sufficient homogeneity. The larger the deviation of the distribution, the larger is the likelihood of inhomogeneity which may cause a malfunction.

The device for determination of a measure for the homogeneity of the lung and/or the data input unit and/or the calculation and control unit may be part of stand-alone EIT device or may be part of an external device, such as a personal computer, a patient monitor or a data processing system of a hospital.

The device for determination of a measure for the homogeneity of the lung may interact with another medical device, such as a ventilator or anaesthetic apparatus, and may form a medical technology system.

In a beneficial embodiment of the device the calculation and control unit is configured to determine for each pixel a deviation value representing the deviation of the impedance amplitude value from the fitted value.

The deviation may for example be determined as the ratio between the impedance amplitude value and the fitted value at the same end-expiratory impedance value.

The deviation may alternatively be determined by the difference between the impedance amplitude value and the fitted value at the same end-expiratory impedance value, the square of the difference or by the logarithm of the ratio between the impedance amplitude value and the fitted value at the same end-expiratory impedance value (log (ΔZ/ΔZ_{ideal})).

The deviation may be a measure of the lung inhomogeneity.

The closer the ratio between the impedance amplitude value and the fitted value is to 1.0, the closer are the respective impedance amplitude values to the fitted values. Hence, the more pixels have a ratio close to 1.0, the greater is the likelihood of an unaffected lung. A great homogeneity is suggestive of a healthy lung. A narrow distribution of ratio values around 1.0 reflects activity of healthy lung tissue during normal breathing.

Alternatively or additionally, the coefficient of determination R² may be used as a criterion for the lung inhomogeneity.

The calculation and control unit may be configured to determine a histogram showing the number of pixels with deviation values in certain intervals for each of the intervals for all pixels of at least one lung.

The distribution of the histogram may give an indication about the status of the lung. The longer the tail on the right side of the value 1.0, the greater the inhomogeneity of the lung and the greater the likelihood of a damage.

The calculation and control unit may be configured to determine an amount of pixels for which the deviation value is larger than a predetermined threshold and/or outside a predetermined region, in particular a predefined region around the fit function, such as a confidence interval.

The amount of pixels may be given by the absolute number or by a percentage.

A threshold may be defined by a cut-off for values which are deemed to be representative for a pathologic condition.

A threshold may be defined by a value, below which a certain percentage (for example 95 %) of deviations of all pixels for a reference patient or for a reference group of, in particular healthy, patients is present.

The borders of the confidence interval or the number of pixels above the threshold may be used as a measure for the homogeneity or the inhomogeneity of the lung.

In an advantageous embodiment the device comprises an output unit and the output unit is configured to use the control signal to provide or output an output signal for displaying a representation of the data.

The output unit may be part of an EIT device or may be part of an external device.

The output unit may display a graphical representation associating the impedance amplitude values and/or fitted amplitude values with the end-expiratory impedance values (EELI) for each pixel, for example a two dimensional graph showing impedance amplitude values ΔZ and fitted values in dependency of end-expiratory impedance values.

The output unit may alternatively or additionally display a regional distribution of end-expiratory impedance values, impedance amplitude values and/or fitted values over at least one lung.

The output unit may alternatively or additionally display a histogram of numbers of pixels being associated with a deviation value within a certain interval, in particular an interval of ratios of impedance amplitude values and fitted amplitude values.

The output unit may alternatively or additionally display an amount of pixels for which a deviation value is larger than a predetermined threshold and/or which are outside a predetermined region.

Without calibration, the absolute values of the impedance amplitude values and the end-expiratory impedance values do not correspond to a certain volume in ml. As there is primarily an interest in the pattern of deviation value distribution, the impedance amplitude values may be normalized over all pixels for at least one lung to obtain a normalized impedance amplitude values for all pixels of at least one lung.

Normalization may be achieved by dividing the impedance amplitude values by the maximum value, in particular for each lung separately.

Alternatively or additionally the end-expiratory impedance values may be normalized over all pixels for at least one lung to obtain a normalized end-expiratory impedance values for all pixels of at least one lung.

The normalized values range up to 1.

In a preferred embodiment the device is configured to obtain, to determine and/or to display reference data, for example EIT data of reference patients, a reference distribution of impedance amplitude values, fitted values and/or a reference histogram.

Preferably, reference data can be determined on the basis of electrical impedance tomography data of reference patients. Reference patients may be patients with a healthy lung. Reference data may be obtained by determining average values for a number of reference patients.

The calculation and control unit may be configured to independently determine data for each of the lungs.

A method for determination of difference parameters based on electrical impedance tomography (EIT) data according to the invention, preferably performed on a device as described above, comprises the following steps.

EIT data are provided from at least one region of at least one lung of a living being over an observation period.

Impedance values are determined over the observation period for each pixel of the at least one region of at least one lung.

An impedance amplitude value and an end-expiratory impedance value are determined for each pixel of at least one lung.

The impedance amplitude value (ΔZ) is associated with the end-expiratory impedance values (EELI) for each pixel. Data are determined on the basis of this information.

A control signal on the basis of the data is generated.

In particular, a calculation and control unit analyses if the data fulfil a predetermined criterion. The data may be compared with a predetermined criterion, such as a predetermined data pattern, a predetermined number or a predetermined range.

A function fit may be performed, for example a linear regression, of the impedance amplitude values in dependency of the end-expiratory impedance values for at least one lung. A fitted function may be obtained, for example a linear function ΔZ_{ideal} = α EELI + β, for at least one lung.

Data representative of a distribution of impedance amplitude values with respect to the fitted values may be determined. A control signal on the basis of the data may be produced.

An EIT-based statistical lung strain distribution may be defined as a deviation from a linear relationship between impedance amplitude values and end-expiratory impedance values.

This can be determined on the level of individual pixels.

Assuming that all pixels corresponding to areas having a healthy ventilation have a linear relationship between impedance amplitude values and end-expiratory impedance values, a plot of impedance amplitude values as a function of end-expiratory impedance values would lie on a straight line for all pixels.

If a larger part of the lung shows healthy ventilation, an ideal ratio between impedance amplitude values and end-expiratory impedance values can be determined from a linear regression of all pixels. Impedance amplitude values lying on this fitted function indicate a constant strain distribution within the lung.

For each pixel a deviation value representing the deviation of the impedance amplitude value from the fitted value may be determined.

To quantify the deviation of each pixel from its ideal strain, a ratio between the measured impedance amplitude value and the fitted function for the same end-expiratory impedance value may be calculated. This may result in a number of deviation values which have a certain statistical distribution.

In a healthy lung the median of the values is close to 1.0. Since the impedance amplitude value is always larger than zero, so is the deviation.

An amount of pixels may be determined for which the deviation value is larger than a predetermined threshold and/or outside a predetermined region.

An output signal may be provided or output for displaying the data. In particular an output signal showing a two dimensional graph of impedance amplitude values and a fitted function in dependency of end expiratory values may be displayed. Additionally or alternatively at least one histogram showing amounts of deviation values may be displayed.

A cut-off may be defined based on the measurements in healthy volunteers. This threshold is necessary since even in healthy lungs, some fluctuations in impedance amplitude value and end-expiratory impedance values will be observed that will lead to deviation values being unequal 1.0.

Data for each of the lungs and/or reference data may be determined and/or displayed independently.

The data determined on the basis of the impedance amplitude values and the associated end-expiratory impedance values for each pixel may be compared with a respective distribution of reference amplitude values and/or deviation values obtained from healthy patients. Depending on the comparison, conclusions about a lung disease or lung malfunction may be drawn, even in an early stage. The device and the method according to the invention may therefore provide a diagnostic tool.

The object of the invention is also solved by a computer program product directly loadable into the internal memory of a computer, in particular into a calculation and control unit of a device as described above and/or an EIT device, wherein the computer program product comprises software code portions for performing the steps of a method as described above when said product is run on a computer.

The invention will now be described with reference to preferred embodiments and the drawings, which show:
- Fig. 1: a schematic presentation of a device for determination of a measure for the homogeneity of the lung based on electrical impedance tomography (EIT) data;
- Fig. 2a: a schematic representation of EIT images during an observation period for a first exemplary patient;
- Fig. 2b: an example for a change of the measured impedances for one pixel during an observation period;
- Fig. 3: a graphical representation of impedance amplitude values in dependence of respective end-expiratory impedance values for the first exemplary patient;
- Fig. 4: a graphical representation of fitted amplitude values for each pixel of the lungs of the first exemplary patient;
- Fig. 5: a graphical representation of deviation values for each pixel of the lungs of the first exemplary patient;
- Fig. 6a: a graphical representation of impedance amplitude values in dependence of respective end-expiratory impedance values of a reference patient;
- Fig. 6b: a graphical representation of impedance amplitude values in dependence of respective end-expiratory impedance values of a COVID 19 patient;
- Fig. 7a: a graphical representation of deviation values of the reference patient;
- Fig. 7b: a graphical representation of deviation values of the COVID 19 patient;
- Fig. 8: a graphical representation of percentages of pixels with a deviation value larger than a threshold of a group of COVID 19 patients in comparison with reference patients.

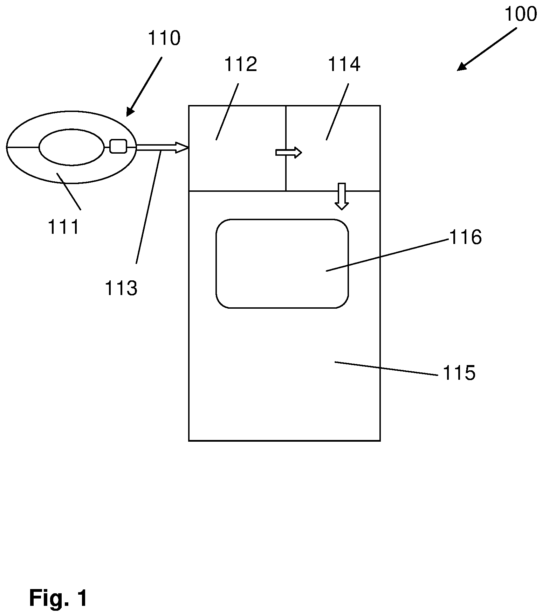

Fig. 1 shows a schematic presentation of an example for a device 100 for determination of a measure for the homogeneity of the lung based on electrical impedance tomography (EIT) data.

The device 100 comprises an EIT apparatus 110 with a belt 111 with electrodes not explicitly shown in the figures. EIT data obtained by the EIT apparatus 110 are provided to a data input unit 112 via a data line 113. A calculation and control unit 114 is connected to the data input unit 112. The calculation and control unit 114 processes EIT data, determines various values representing a measure for the homogeneity of the lung and provides output signals as described below.

These output signals may be provided to an output unit 115, which comprises a display unit 116 for displaying the determined values.

EIT measurements were recorded with the Sentec BB2 (Sentec AG, EIT branch, Landquart, Switzerland) using a textile electrode belt with 32 electrodes. Impedance tomography data was recorded at a sampling rate of 47.68 Hz. Patient-specific ventilation images of breathing-induced impedance changes were calculated in relation to a reference measurement using the manufacturer's imaging algorithm. All calculations were done offline using Matlab R2018b (The MathWorks, Natick, Massachusetts). The images of 32 by 32 pixels created by the manufacturer's imaging algorithm were used to calculate strain images.

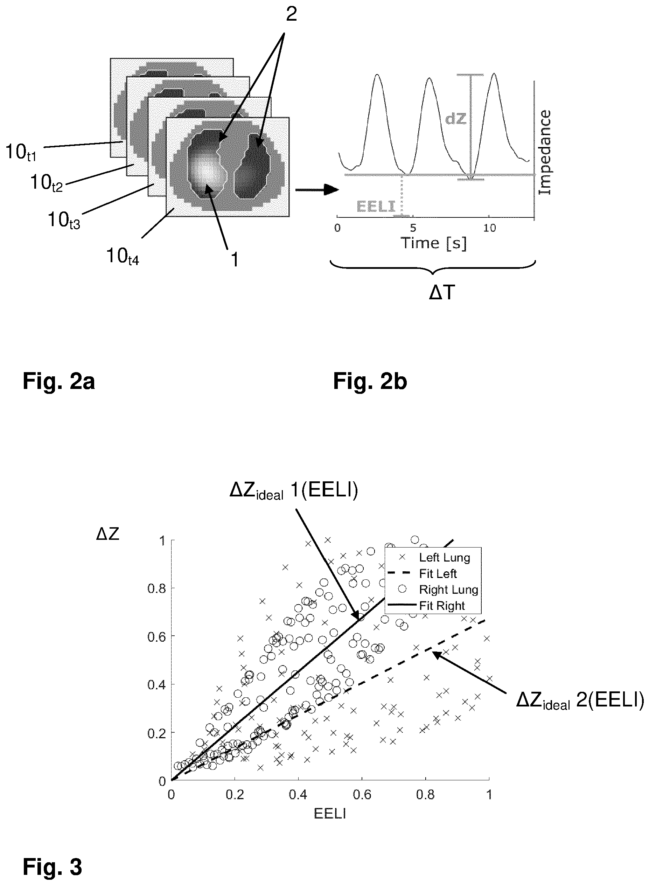

Figure 2a shows a schematic representation of EIT images 10ₜ₁, 10ₜ₂, 10ₜ₃, 10ₜ₄ for different points of time during an observation period ΔT for a first exemplary patient. The EIT image is based on impedance values at pixels 1 of each of the lungs 2 and shows impedance values measured for each pixel in an observation plane defined by the position of the belt 111 on the patient. Each pixel of the EIT mage is coloured according to the impedance value of the pixel.

Figure 2b shows an example for a change of the measured impedance values for one pixel 1 during an observation period ΔT.

An impedance amplitude value ΔZ is determined as the maximal difference between a maximum and a subsequent minimum of the measured impedance during the observation period ΔT.

An end-expiratory impedance value EELI may be determined as the average minimum value of the impedance during the observation period ΔT.

For each pixel 1 of both lungs 2 the impedance amplitude value ΔZ and the end-expiratory impedance values EELI is determined.

Figure 3 shows a graphical representation of impedance amplitude values ΔZ in dependence of respective end-expiratory impedance values EELI for the first exemplary patient.

Since there is primarily an interest in the pattern, impedance amplitude values ΔZ and end-expiratory impedance values EELI were normalized to range up to 1.0. The impedance amplitude values ΔZ and the end-expiratory impedance values EELI were divided by their respective maxima.

The impedance amplitude values ΔZ were normalized for both lungs separately, dividing ΔZ for each lung 2 by the maximum value in that lung 2.

The normalized impedance amplitude values ΔZ for each pixel are plotted against their respective normalized end-expiratory impedance values EELI.

A function fit, in this case a linear regression ΔZ = α · EELI, is performed for both lungs separately to obtain fitted functions ΔZ_{ideal}1 (EELI) and ΔZ_{ideal}2 (EELI) for each of the both lungs 2. The values ΔZ_{ideal}1, ΔZ_{ideal}2 are the values that would correspond to the specific end-expiratory impedance values if there was a constant strain in each lung.

Similarly, a function fit ΔZ = α · EELI + β may be performed, wherein EELI could be determined/rescaled in a way that eliminates the offset β. The linear relationship between ΔZ and EELI mirrors a linear relationship between change in lung volume dV and the resting lung volume V₀.

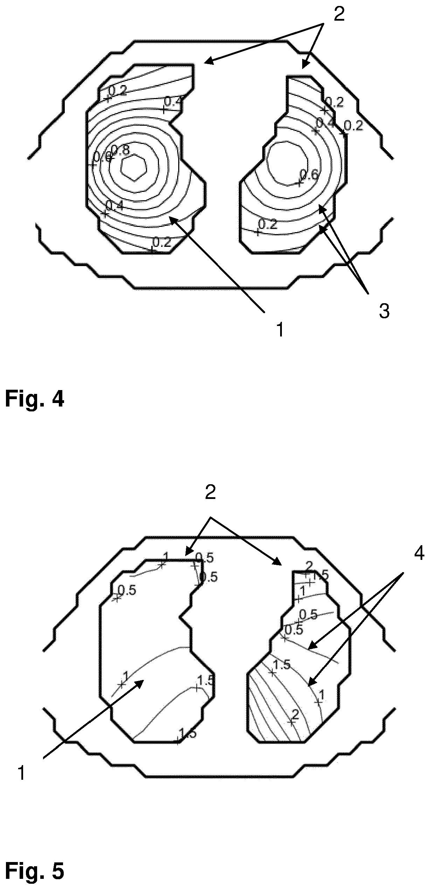

ΔZ_{ideal}1, ΔZ_{ideal}2 for each pixel of the respective lungs 2 of the exemplary patient is shown in figure 4. Each pixel 1 of the observation plane assigned to one of the lungs 2 is coloured according to the value ΔZ_{ideal}1 or ΔZ_{ideal}2 for the respective value EELI of the respective pixel.

Figure 4 shows a graphical representation of fitted amplitude values for each pixel of the lungs of the first exemplary patient, wherein equal values of the amplitude values are shown as level lines **3.**

It can be seen that a linear fit represents a reasonable model of a normal behaviour of the lungs **2.**

Values with large ideal amplitudes ΔZ_{ideal} are located at pixels in the centre of the lung 2, whereas smaller values can be found in the boundary regions of the lungs **2.**

For each pixel a deviation value Strain_{EIT} may be determined representing the deviation of the impedance amplitude value ΔZ from the fitted value ΔZ_{ideal}.

In this example, the deviation value Strain_{EIT} is obtained by the ratio ΔZ/ΔZ_{ideal} between the impedance amplitude value ΔZ and the fitted value ΔZ_{ideal} at the same end-expiratory impedance value EELI for each pixel 1 of each lung **2.**

Figure 5 shows a graphical representation of the regional distribution of the deviation values Strain_{EIT}, ΔZ/ΔZ_{ideal}1 and ΔZ/ΔZ_{ideal}2, for the first exemplary patient. Each pixel 1 of the observation plane belonging to one of the lungs 2 is assigned to the value ΔZ/ΔZ_{ideal}1 or ΔZ/ΔZ_{ideal}2 for the end-expiratory value EELI of the respective pixel. Equal values of the respective deviation values Strain_{EIT}, ΔZ/ΔZ_{ideal}1 and ΔZ/ΔZi_{deal}2, are shown as level lines 4 for each of the lungs 2.

As can be seen in comparison with Figure 4, the deviation values are distributed differently from the fitted values ΔZ_{ideal}.

Regions of the lungs may be identified, where the deviation values ΔZ/ΔZ_{ideal}1 deviate more or less from an ideal and undisturbed deviation value of 1.0. These regions may be considered to have a disturbed lung strain.

Hence, areas with high strain within the lung as well as patients with unphysiological lung inhomogeneity can be identified.

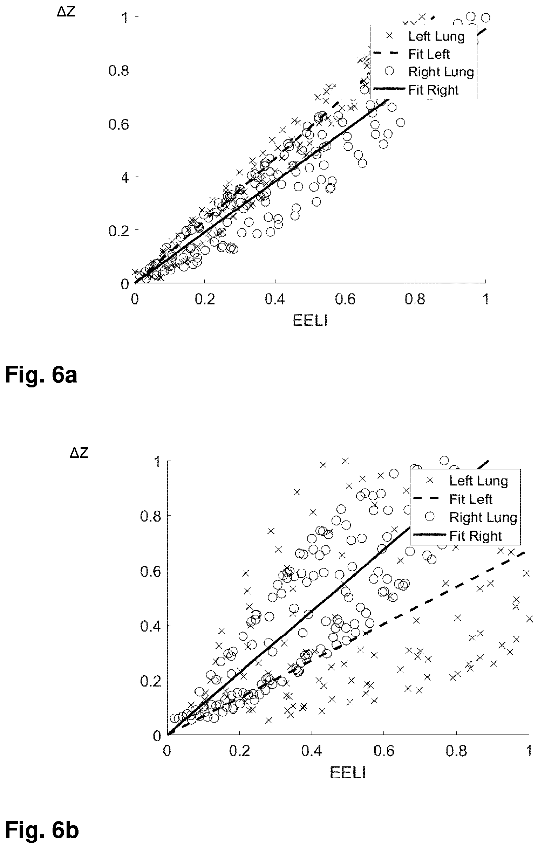

Figure 6a shows a graphical representation of normalized impedance amplitude values ΔZ in dependence of respective normalized end-expiratory impedance values EELI of a reference patient, whereas Figure 6b shows a graphical representation of normalized impedance amplitude values ΔZ in dependence of respective end-expiratory impedance values EELI of a COVID 19 patient.

Both representations show measured pixel values and fit functions for both lungs separately.

Impedance amplitude values ΔZ and respective end-expiratory impedance values EELI of the reference patient were obtained by an EIT measurement at a healthy volunteer.

For COVID-19 patients, two consecutive EIT measurements were performed 3 days apart. For healthy volunteers, only one measurement was recorded. All measurements may be recorded in sitting, supine, left and right lateral position. Examples shown in this application are based only from EIT recordings in the supine position.

Lung strain resulting from increased respiratory drive may inflict high energy loads on lung tissue thereby increasing the risk for patient self-inflicted lung injury. Such high lung strain may be contributing to respiratory failure in COVID-19 patients. Respiratory failure due to COVID-19 pneumonia may progress rapidly to an acute respiratory distress syndrome (ARDS) like clinical picture with an extremely inhomogeneously damaged lung. These patients often develop, even in the early phase, a severe hypoxemia and a pathologic respiratory drive with high respiratory load. During the course of the disease this increased respiratory drive, which induces high tidal strain and energy loads on the vulnerable lung tissue, increases the risk for Patient Self-Inflicted Lung Injury.

Even in a healthy lung, strain is not zero. Instead, the volume change during ventilation in a region is compatible with the total lung volume in that region resulting in a physiological level of strain. Furthermore, even in a healthy lung, some pixels will deviate from the linear relationship. Therefore a typical distribution of these values needs to be defined from measurements in healthy volunteers.

Accordingly, impedance amplitude values ΔZ are not exactly on the fitted linear line.

In a study including ten healthy volunteers aged 32 ± 8 years and ten COVID-19 patients aged 55 ± 21 years, overall a good correlation, with a mean R² valueof 0.80 for COVID 19 patients and of 0.92 for healthy volunteers was found. R²-can be calculated by R² = 1- sum (ΔZ - ΔZ_{ideal})²/sum(ΔZ - mean (ΔZ))². The lowest R² values were 0.77 in volunteers and 0.29 in patients.

Correlation itself, as indicated by the coefficient of determination R² may be considered as a measure of strain and/or a measure for inhomogeneity. However, information about which pixels show the right ratio ΔZ/EELI of impedance amplitude value ΔZ and end-expiratory impedance values EELI becomes less clear the fewer healthy lung pixels there are. Therefore, information about the regional distribution of strain should be handled with caution in cases of low R².

However, as can be seen in Figure 6b the variation of impedance amplitude value with respect to the fitted function is way greater for the COVID -19 patient than for the healthy reference patient.

Thus, the distribution of normalized impedance amplitude values ΔZ in dependence of respective normalized end-expiratory impedance values EELI may be considered to be a measure of the inhomogeneity of the lung.

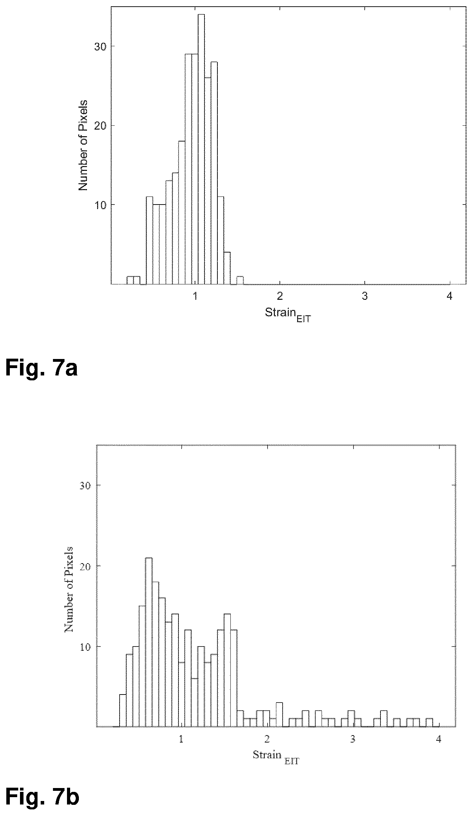

Figure 7a shows a graphical representation of deviation values Strain_{EIT} of the reference patient and Figure 7b shows a graphical representation of deviation values Strain_{EIT} of the COVID-19 patient.

The deviation values Strain_{EIT} were calculated as the ratio of the impedance amplitude values ΔZ and the value of the fitted function ΔZ_{ideal} at the same end-expiratory impedance values EELI.

The mean of these ratios is always 1.0, and in a healthy lung most pixels have deviation values Strain_{EIT} close to 1.0.

Physiologically, the values that are too high are more critical for patients. Alternatively, for example log (ΔZ/ΔZ_{ideal}) coould be determined for all pixels. This consideration provides tails on both sides of the ideal value and allows to set a threshold on both sides of the ideal value. Not only values of ΔZ/ΔZ_{ideal} which are too large, but also values of ΔZ/ΔZ_{ideal} which are too small can be taken into account.

Figures 7a and 7b show histograms of numbers of pixels with a deviation value Strain_{EIT} being in a respective one of equidistant intervals between two deviation values. In this case the intervals have the lengths of 1/10.

As can be seen in Figure 7a for the healthy reference patient as expected there is a maximum at 1 and most of the pixels are at least close to 1.

For the COVID-19 patient the histogram shows a long tail meaning that many pixels have a large deviation value Strain_{EIT}.

A cutoff may be defined based on the measurements in a sample group of healthy volunteers. Assuming that in healthy lung tissue only a few outlier pixels will show unphysiologically large strain, the ratio of impedance amplitude value ΔZ and fitted function values ΔZ_{ideal}, for which 97.5% of all pixels in healthy volunteers have a lower ratio, may be determined as the cutoff for healthy lung strain.

In images of the regional strain EIT distribution (see Figure 5), pixels with values above this threshold show areas of high lung strain.

Furthermore, the number of pixels displaying such high deviation values can be an indicator for total lung strain. Hence, the sum of those pixels can be considered as an indicator for the total strain.

In patients of said study, an average of 21 out of 239 pixels in the lung region showed a strain value larger than 2.11, while this was only the case for an average of 6 pixels out of 242 in the lung region in the healthy volunteers.

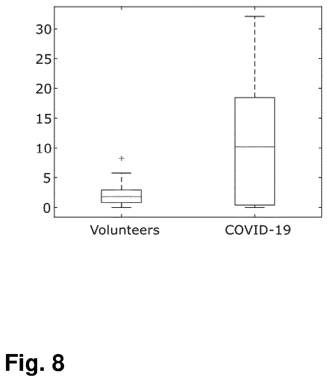

Figure 8 shows a graphical representation in the form of boxplots for percentages of pixels with a deviation value larger than a threshold of a group of COVID 19 patients of the study in comparison with reference patients of the study.

The difference in this number between patients and volunteers is conserved to be statistically significant at a p-value calculated using a two-sample test of 0.028.

p is the probability that the strain values for patients and volunteers come from independent random samples from normal distributions with equal means and equal but unknown variances (null hypothesis). A test statistic t is calculated and p is the probability of observing a test statistic as extreme as, or more extreme than, the observed value under the null hypothesis. The calculation is done with statistical software which uses numerical methods. Here, Matlabs's ttest2 function was used.

The median percentage of pixels larger than 2.11 was 1.8% for healthy volunteers and 10.2% for COVID-19 patients.

## Claims

1. A device (100) for determination of a measure for the homogeneity of the lung based on electrical impedance tomography (EIT) data, the device comprising:
a data input unit (112), receiving the EIT data obtained by an electrical impedance tomography apparatus (110), wherein the data input unit (112) is configured to receive and provide EIT data from at least one region of at least one lung (2) of a living being over an observation period (ΔT);
a calculation and control unit (114) connected to the data input unit,
wherein the calculation and control unit (114) is configured
- to determine impedance values over the observation period (ΔT) for each pixel (1) of the at least one region of at least one lung (2);
- to determine an impedance amplitude value (ΔZ) for each pixel (1) of at least one lung (2);
- to determine an end-expiratory impedance value (EELI) for each pixel of at least one lung;
- to associate the impedance amplitude values (ΔZ) and the end-expiratory impedance values (EELI) individually for each pixel (1);
- to determine data on the basis of the impedance amplitude values (ΔZ) and the associated end-expiratory impedance values (EELI), and
- to generate a control signal on basis of the data,
- in particular to compare the data with a predetermined criterion,
- wherein
the calculation and control unit (114) is configured to perform a function fit of the impedance amplitude values (ΔZ) in dependency of the end-expiratory impedance values (EELI) for at least one lung to obtain a fitted function (ΔZ_{ideal} (EELI)), which is a linear function ΔZ_{ideal} = α EELI + β, for at least one lung.

2. A device according to claim 1, wherein the calculation and control unit (114) is configured to compare the data with a criterion, which criterion has been predetermined on the basis of a control group of patients.

3. A device according to claim 1 or 2, wherein the calculation and control unit is configured to determine for each pixel a deviation value (Strain_{EIT}) representing the deviation of the impedance amplitude value ΔZ from the fitted value (ΔZ_{ideal}), in particular the ratio (ΔZ/ΔZ_{ideal}) between the impedance amplitude value (ΔZ) and the fitted value (ΔZ_{ideal}) at the same end-expiratory impedance value (EELI).

4. A device according to one of the preceding claims, wherein the calculation and control unit (114) is configured to determine a histogram showing the number of pixels with deviation values (Strain_{EIT}) in certain intervals for each of the intervals for all pixels (1) of at least one lung (2).

5. A device according to one of the preceding claims 3 to 5, wherein the calculation and control unit (114) is configured to determine an amount of pixels (1) for which the deviation value (Strain_{EIT}) is larger than a predetermined threshold and/or for which the deviation value (Strain_{EIT}) is outside a predetermined region.

6. A device according to one of the preceding claims, wherein the device comprises an output unit (115) and the output unit (115) is configured to use the control signal to provide or output an output signal for displaying a representation of the data,
in particular to display
- a graph associating the impedance amplitude values (ΔZ) and/or fitted amplitude values (ΔZ_{ideal}) with the end-expiratory impedance values (EELI) for each pixel,
- a regional distribution of end-expiratory impedance values (EELI), impedance amplitude values (ΔZ) and/or fitted values (ΔZ_{ideal}) over at least one lung,
- a histogram of numbers of pixels being associated with a deviation value (Strain_{EIT}) within a certain interval, in particular an interval of ratios of impedance amplitude values (ΔZ) and fitted amplitude values (ΔZ_{ideal})
- a diagram showing an amount of pixels (1) for which a deviation value (Strain_{EIT}) is larger than a predetermined threshold and/or for which a deviation value (Strain_{EIT}) is outside a predetermined region.

7. A device according to one of the preceding claims, wherein the calculation and control unit (114) is configured
to normalize the impedance amplitude values (ΔZ) over all pixels (1) for at least one lung (2) and to obtain a normalized amplitude value (ΔZ) for all pixels of at least one lung and/or
to normalize the end-expiratory impedance values (EELI) over all pixels (1) for at least one lung (2) and to obtain a normalized end-expiratory impedance value (EELI) for each pixel (1) of at least one lung (2).

8. A device according to one of the preceding claims which is configured to determine and/or display reference data, for example a reference distribution of impedance amplitude values (ΔZ) and fitted values (ΔZ_{ideal}) or a reference histogram, for example on the basis of electrical impedance tomography (EIT) data of reference patients.

9. A device according to one of the preceding claims wherein the calculation and control unit (114) is configured to determine data independently for each of the lungs (2).

10. A method for determination of a measure for the homogeneity of the lung based on electrical impedance tomography (EIT) data, with a device according to one of the preceding claims, the method comprising:
- providing EIT data from at least one region of at least one lung (2) of a living being over an observation period (ΔT) ;
- determining impedance values over the observation period for each pixel (1) of the at least one region of at least one lung (2);
- determining an impedance amplitude value (ΔZ) for each pixel (1) of at least one lung (2);
- determining an end-expiratory impedance value (EELI) for each pixel (1) of at least one lung (2);
- associating the impedance amplitude values (ΔZ) and the end-expiratory impedance values (EELI) individually for each pixel (1)
- determining data on the basis of the impedance amplitude values (ΔZ) and the associated end-expiratory impedance values (EELI);
- generating a control signal on the basis of the data,
- in particular, comparing the data with a predetermined criterion
- performing a function fit of the impedance amplitude values (ΔZ) in dependency of the end-expiratory impedance values (EELI) for at least one lung to obtain a fitted function (ΔZ_{ideal}(EELI)), in particular a linear function ΔZ_{ideal} = α EELI + β, for at least one lung (2) and determining data representative of a distribution of impedance amplitude values (ΔZ) with respect to the fitted values (ΔZ_{ideal}).

11. A method according to claim 10, comprising the step of predetermining a criterion on the basis of a control group of patients, and preferably saving the criterion to a data storage.

12. A method according to claim 10 or 11, comprising the step of determining for each pixel a deviation value (Strain_{EIT}) representing the deviation of the impedance amplitude value (ΔZ) from the fitted value (ΔZ_{ideal}), in particular the ratio (ΔZ/ΔZ_{ideal}) between impedance amplitude values (ΔZ) and the fitted value (ΔZ_{ideal}) at the same end-expiratory impedance value (EELI).

13. A method according to claim 12, comprising the step of determining an amount of pixels (1) for which the deviation value (Strain_{EIT}) is larger than a predetermined threshold and/or for which the deviation value (Strain_{EIT}) is outside a predetermined region.

14. A method according to one of claims 10 to 13, comprising the step of providing or outputting an output signal for displaying a representation of the data, in particular displaying the output signal.

15. A method according to one of claims 10 to 14, comprising the step of determining data for each of the lungs (2) and/or comprising the step of determining reference data.

16. Computer program product directly loadable into a computer and/or for running on the computer, in particular into a calculation and control unit of a devices according to claims 1-9 and/or an EIT device, wherein the computer program product comprises software code portions for performing the steps of a method according to one of claims 10-15.

## Patentansprüche

1. Vorrichtung (100) zur Bestimmung eines Maßes für die Homogenität der Lunge auf der Grundlage von Daten der elektrischen Impedanztomographie (EIT), wobei die Vorrichtung umfasst:
eine Dateneingabeeinheit (112), die die von einem elektrischen Impedanztomographiegerät (110) erhaltenen EIT-Daten empfängt, wobei die Dateneingabeeinheit (112) so konfiguriert ist, dass sie EIT-Daten aus mindestens einem Bereich mindestens einer Lunge (2) eines Lebewesens über einen Beobachtungszeitraum (ΔT) empfängt und bereitstellt;
eine mit der Dateneingabeeinheit verbundene Berechnungs- und Steuereinheit (114),
wobei die Berechnungs- und Steuereinheit (114) konfiguriert ist
- Impedanzwerte über den Beobachtungszeitraum (ΔT) für jedes Pixel (1) mindestens eines Bereichs mindestens einer Lunge (2) zu bestimmen;
- einen Impedanzamplitudenwert (ΔZ) für jedes Pixel (1) mindestens einer Lunge (2) zu bestimmen;
- einen end-exspiratorischen Impedanzwert (EELI) für jedes Pixel mindestens einer Lunge zu bestimmen;
- die Impedanzamplitudenwerte (ΔZ) und die end-exspiratorischen Impedanzwerte (EELI) jedem Pixel (1) individuell zuzuordnen;
- Daten auf der Grundlage der Impedanzamplitudenwerte (ΔZ) und der zugeordneten end-exspiratorischen Impedanzwerte (EELI) zu bestimmen, und
- auf der Grundlage der Daten ein Steuersignal zu erzeugen,
- insbesondere die Daten mit einem vorgegebenen Kriterium zu vergleichen,
wobei
die Berechnungs- und Steuereinheit (114) dazu konfiguriert ist, eine Funktionsanpassung der Impedanzamplitudenwerte (ΔZ) in Abhängigkeit von den end-exspiratorischen Impedanzwerten (EELI) für mindestens eine Lunge durchzuführen, um eine angepasste Funktion (ΔZ_{ideal} (EELI)) zu erhalten, die eine lineare Funktion ΔZ_{ideal} = α EELI + β für mindestens eine Lunge ist.

2. Vorrichtung nach Anspruch 1, wobei die Berechnungs- und Steuereinheit (114) so konfiguriert ist, dass sie die Daten mit einem Kriterium vergleicht, das auf der Grundlage einer Kontrollgruppe von Patienten vorgegeben ist.

3. Vorrichtung nach Anspruch 1 oder 2, wobei die Berechnungs- und Steuereinheit so konfiguriert ist, dass sie für jedes Pixel einen Abweichungswert (Strain_{EIT}) bestimmt, der die Abweichung des Impedanzamplitudenwerts ΔZ vom angepassten Wert (ΔZ_{ideal}) darstellt, insbesondere das Verhältnis (ΔZ/ΔZ_{ideal}) zwischen dem Impedanzamplitudenwert (ΔZ) und dem angepassten Wert (ΔZ_{ideal}) bei gleichem end-exspiratorischem Impedanzwert (EELI).

4. Vorrichtung nach einem der vorstehenden Ansprüche, wobei die Berechnungs- und Steuereinheit (114) dazu ausgelegt ist, ein Histogramm zu ermitteln, das die Anzahl der Pixel mit Abweichungswerten (Strain_{EIT}) in bestimmten Intervallen für jedes der Intervalle für alle Pixel (1) mindestens einer Lunge (2) anzeigt.

5. Vorrichtung nach einem der vorstehenden Ansprüche 3 bis 5, wobei die Berechnungs- und Steuereinheit (114) so konfiguriert ist,
dass sie eine Anzahl von Pixeln (1) bestimmt, für die der Abweichungswert (Strain_{EIT}) größer als ein vorbestimmter Schwellenwert ist und/oder für die der Abweichungswert (Strain_{EIT}) außerhalb eines vorbestimmten Bereichs liegt.

6. Vorrichtung nach einem der vorstehenden Ansprüche, wobei die Vorrichtung eine Ausgabeeinheit (115) umfasst und die Ausgabeeinheit (115) so konfiguriert ist, dass sie das Steuersignal verwendet, um ein Ausgangssignal zum Anzeigen einer Darstellung der Daten bereitzustellen oder auszugeben, insbesondere zum Anzeigen
- eines Diagramms, das die Impedanzamplitudenwerte (ΔZ) und/oder die angepassten Amplitudenwerte (ΔZ_{ideal}) den end-exspiratorischen Impedanzwerten (EELI) für jedes Pixel zuordnet,
- einer regionalen Verteilung der end-exspiratorischen Impedanzwerte (EELI), der Impedanzamplitudenwerte (ΔZ) und/oder der angepassten Werte (ΔZ_{ideal}) über mindestens eine Lunge,
- eines Histogramms der Anzahl von Pixeln, die mit einem Abweichungswert (Strain_{EIT}) innerhalb eines bestimmten Intervalls, insbesondere eines Intervalls von Verhältnissen von Impedanzamplitudenwerten (ΔZ) und angepassten Amplitudenwerten (ΔZ_{ideal}), verbunden sind,
- eines Diagramm, das eine Anzahl von Pixeln (1) zeigt, für die ein Abweichungswert (Strain_{EIT}) größer als ein vorbestimmter Schwellenwert ist und/oder für die ein Abweichungswert (Strain_{EIT}) außerhalb eines vorbestimmten Bereichs liegt.

7. Vorrichtung nach einem der vorstehenden Ansprüche, wobei die Berechnungs- und Steuereinheit (114) konfiguriert ist, die Impedanzamplitudenwerte (ΔZ) über alle Pixel (1) für mindestens eine Lunge (2) zu normalisieren und einen normalisierten Amplitudenwert (ΔZ) für alle Pixel mindestens einer Lunge zu erhalten,
und/oder
die end-exspiratorischen Impedanzwerte (EELI) über alle Pixel (1) für mindestens eine Lunge (2) zu normalisieren und einen normalisierten end-exspiratorischen Impedanzwert (EELI) für jedes Pixel (1) mindestens einer Lunge (2) zu erhalten.

8. Vorrichtung nach einem der vorstehenden Ansprüche, die dazu konfiguriert ist, Referenzdaten, beispielsweise eine Referenzverteilung von Impedanzamplitudenwerten (ΔZ) und angepassten Werten (ΔZ_{ideal}) oder ein Referenzhistogramm, beispielsweise auf der Grundlage von elektrischen Impedanztomographie (EIT)-Daten von Referenzpatienten, zu bestimmen und/oder anzuzeigen.

9. Vorrichtung nach einem der vorstehenden Ansprüche, wobei die Berechnungs- und Steuereinheit (114) so konfiguriert ist, dass sie Daten für jede der Lungen (2) unabhängig voneinander bestimmt.

10. Verfahren zur Bestimmung eines Maßes für die Homogenität der Lunge auf der Grundlage von elektrischen Impedanztomographie (EIT)-Daten, mit einer Vorrichtung nach einem der vorstehenden Ansprüche, wobei das Verfahren umfasst:
- Bereitstellen von EIT-Daten aus mindestens einem Bereich mindestens einer Lunge (2) eines Lebewesens über einen Beobachtungszeitraum (ΔT);
- Bestimmen von Impedanzwerten über den Beobachtungszeitraum für jedes Pixel (1) mindestens eines Bereichs mindestens einer Lunge (2);
- Bestimmen eines Impedanzamplitudenwerts (ΔZ) für jedes Pixel (1) der mindestens einen Lunge (2);
- Bestimmen eines end-exspiratorischen Impedanzwerts (EELI) für jedes Pixel (1) der mindestens einen Lunge (2);
- individuelles Zuordnen der Impedanzamplitudenwerte (ΔZ) und der end-exspiratorischen Impedanzwerte (EELI) zu jedem Pixel (1);
- Ermitteln von Daten auf der Grundlage der Impedanzamplitudenwerte (ΔZ) und der zugeordneten end-exspiratorischen Impedanzwerte (EELI);
- Erzeugen eines Steuersignals auf der Grundlage der Daten,
- insbesondere Vergleichen der Daten mit einem vorgegebenen Kriterium
- Durchführen einer Funktionsanpassung der Impedanzamplitudenwerte (ΔZ) in Abhängigkeit von den end-exspiratorischen Impedanzwerten (EELI) für mindestens eine Lunge, um eine angepasste Funktion (ΔZ_{ideal} (EELI)) zu erhalten, insbesondere einer linearen Funktion ΔZ_{ideal} = α EELI + β, für mindestens eine Lunge (2) und Bestimmen von Daten, die eine Verteilung von Impedanzamplitudenwerten (ΔZ) in Bezug auf die angepassten Werte (ΔZ_{ideal}) repräsentieren.

11. Verfahren nach Anspruch 10, umfassend den Schritt des Vorbestimmens eines Kriteriums auf der Grundlage einer Kontrollgruppe von Patienten und vorzugsweise des Speicherns des Kriteriums in einem Datenspeicher.

12. Verfahren nach Anspruch 10 oder 11, umfassend den Schritt des Bestimmens für jedes Pixel eines Abweichungswertes (Strain_{EIT}), der die Abweichung des Impedanzamplitudenwertes (ΔZ) vom angepassten Wert (ΔZ_{ideal}) darstellt, insbesondere das Verhältnis (ΔZ/ΔZ_{ideal}) zwischen Impedanzamplitudenwerten (ΔZ) und dem angepassten Wert (ΔZ_{ideal}) bei gleichem end-exspiratorischem Impedanzwert (EELI).

13. Verfahren nach Anspruch 12, umfassend den Schritt des Bestimmens einer Anzahl von Pixeln (1), für die der Abweichungswert (Strain_{EIT}) größer als ein vorbestimmter Schwellenwert ist und/oder für die der Abweichungswert (Strain_{EIT}) außerhalb eines vorbestimmten Bereichs liegt.

14. Verfahren nach einem der Ansprüche 10 bis 13, umfassend den Schritt des Bereitstellens oder Ausgebens eines Ausgangssignals zum Anzeigen einer Darstellung der Daten, insbesondere zum Anzeigen des Ausgangssignals.

15. Verfahren nach einem der Ansprüche 10 bis 14, umfassend den Schritt des Bestimmens von Daten für jede der Lungen (2) und/oder umfassend den Schritt des Bestimmens von Referenzdaten.

16. Computerprogrammprodukt, das direkt in einen Computer und/oder zur Ausführung auf dem Computer, insbesondere in eine Rechen- und Steuereinheit einer Vorrichtung nach den Ansprüchen 1 bis 9 und/oder einer EIT-Vorrichtung, ladbar ist, wobei das Computerprogrammprodukt Software-Codeteile zum Ausführen der Schritte eines Verfahrens nach einem der Ansprüche 10 bis 15 umfasst.

## Revendications

1. Dispositif (100) pour la détermination d'une mesure de l'homogénéité du poumon sur la bade de données de tomographie d'impédance électrique (EIT), ledit dispositif comprenant :
une unité d'entrée de données (112), recevant les données EIT obtenues par un appareil de tomographie d'impédance électrique (110), ladite unité d'entrée de données (112) étant configurée pour recevoir et fournir des données EIT provenant d'au moins une région d'au moins un poumon (2) d'un être vivant pendant une période d'observation (ΔT) ;
une unité de calcul et de contrôle (114) connectée à l'unité d'entrée des données,
dans laquelle l'unité de calcul et de contrôle (114) est configurée pour
- déterminer des valeurs d'impédance sur la période d'observation (ΔT) pour chaque pixel (1) de ladite au moins une région d'au moins un poumon (2) ;
- déterminer une valeur d'amplitude d'impédance (ΔZ) pour chaque pixel (1) d'au moins un poumon (2) ;
- déterminer une valeur d'impédance de fin d'expiration (EELI) pour chaque pixel d'au moins un poumon ;
- associer, pour chaque pixel (1), la valeur d'amplitude d'impédance (ΔZ) et la valeur d'impédance de fin d'expiration (EELI) correspondante;
- déterminer des données sur la base des valeurs d'amplitude d'impédance (ΔZ) et des valeurs d'impédance de fin d'expiration (EELI) associées, et
- générer un signal de commande sur la base de ces données,
- en particulier pour comparer les données à un critère prédéterminé,
dans lequel
l'unité de calcul et de contrôle (114) est configurée pour réaliser un ajustement fonctionnel des valeurs d'amplitude d'impédance (ΔZ) en fonction des valeurs d'impédance de fin d'expiration (EELI), pour au moins un poumon, afin d'obtenir une fonction ajustée (ΔZ_{(idéal)} (EELI)), ladite fonction étant une fonction linéaire ΔZ_{i-déal}= α EELI + β.

2. Dispositif selon la revendication 1, dans lequel l'unité de calcul et de contrôle (114) est configurée pour comparer les données à un critère, ce critère ayant été prédéterminé sur la base d'un groupe contrôle de patients.

3. Dispositif selon la revendication 1 ou 2, dans lequel l'unité de calcul et de contrôle est configurée pour déterminer, pour chaque pixel, une valeur de déviation (Strain_{(EIT})) représentant l'écart de la valeur d'amplitude d'impédance ΔZ par rapport à la valeur ajustée (ΔZ_{idéal}), en particulier le rapport (ΔZ/ΔZ_{idéal}) entre la valeur d'amplitude de l'impédance (ΔZ) et la valeur ajustée (ΔZ_{idéal}) à la même valeur d'impédance de fin d'expiration (EELI).

4. Dispositif selon l'une des revendications précédentes, dans lequel l'unité de calcul et de contrôle (114) est configurée pour déterminer un histogramme représentant le nombre de pixels ayant des valeurs déviation (Strain_{(EIT})) dans certains intervalles pour chacun des intervalles pour tous les pixels (1) d'au moins un poumon (2).

5. Dispositif selon l'une des revendications précédentes 3 à 5, dans lequel l'unité de calcul et de contrôle (114) est configurée pour déterminer une quantité de pixels (1) pour lesquels la valeur de déviation (Strain_{(EIT}) ) est supérieure à un seuil prédéterminé et/ou pour lesquels la valeur de déviation (Strain_{(EIT}) ) se situe en dehors d'une plage prédéterminée.

6. Dispositif selon l'une des revendications précédentes, dans lequel le dispositif comprend une unité de sortie (115) configurée pour utiliser le signal de commande afin de fournir ou d'émettre un signal de sortie pour afficher une représentation des données,
en particulier pour afficher
- un graphe associant les valeurs d'amplitude d'impédance (ΔZ) et/ou les valeurs d'amplitude ajustées (ΔZ_{idéal}) aux valeurs d'impédance de fin d'expiration (EELI) pour chaque pixel,
- une distribution régionale des valeurs d'impédance de fin d'expiration (EELI), des valeurs d'amplitude d'impédance (ΔZ) et/ou des valeurs ajustées (ΔZ_{idéal}) sur au moins un poumon,
- un histogramme des nombres de pixels associés à une valeur de déviation (Strain_{(EIT}) ) dans un certain intervalle, en particulier un intervalle de rapports entre les valeurs d'amplitude d'impédance (ΔZ) et les valeurs d'amplitude ajustées (ΔZ_{idéal)},
- un diagramme montrant une quantité de pixels (1) pour lesquels une valeur de déviation (Strain_{(EIT}) ) est supérieure à un seuil prédéterminé et/ou pour lesquels une valeur de déviation (Strain_{(EIT}) ) se situe en dehors d'une plage prédéterminée.

7. Dispositif selon l'une des revendications précédentes, dans lequel l'unité de calcul et de contrôle (114) est configurée pour normaliser les valeurs d'amplitude d'impédance (ΔZ) sur l'ensemble des pixels (1) d'au moins un poumon (2) et obtenir une valeur d'amplitude normalisée (ΔZ) pour l'ensemble des pixels d'au moins un poumon
et/ou
de normaliser les valeurs d'impédance de fin d'expiration (EELI) sur tous les pixels (1) d'au moins un poumon (2) et d'obtenir une valeur d'impédance de fin d'expiration normalisée (EELI) pour chaque pixel (1) d'au moins un poumon (2).

8. Dispositif selon l'une des revendications précédentes, configuré pour déterminer et/ou afficher des données de référence, par exemple une distribution de référence des valeurs d'amplitude d'impédance (ΔZ) et des valeurs ajustées (ΔZ_{(idéal}) ) ou un histogramme de référence, par exemple sur la base de données de tomographie d'impédance électrique (EIT) de patients de référence.

9. Dispositif selon l'une des revendications précédentes, dans lequel l'unité de calcul et de contrôle (114) est configurée pour déterminer les données pour chacun des poumons (2) indépendamment.

10. Procédé de détermination d'une mesure d'homogénéité du poumon à partir de données de tomographie d'impédance électrique (EIT), avec un dispositif selon l'une des revendications précédentes, le procédé comprenant :
- fournir des données EIT d'au moins une région d'au moins un poumon (2) d'un être vivant sur une période d'observation (ΔT) ;
- déterminer les valeurs d'impédance sur la période d'observation pour chaque pixel (1) d'au moins une région d'au moins un poumon (2) ;
- déterminer une valeur d'amplitude d'impédance (ΔZ) pour chaque pixel (1) d'au moins un poumon (2) ;
- déterminer une valeur d'impédance de fin d'expiration (EELI) pour chaque pixel (1) d'au moins un poumon (2) ;
- associer les valeurs d'amplitude d'impédance (ΔZ) et les valeurs d'impédance de fin d'expiration (EELI) individuellement pour chaque pixel (1)
- déterminer des données sur la base des associations de valeurs d'amplitude d'impédance (ΔZ) et des valeurs d'impédance de fin d'expiration (EELI);
- générer un signal de commande sur la base des données,
- en particulier, en comparant les données à un critère prédéterminé
- effectuer un ajustement fonctionnel des valeurs d'amplitude d'impédance (ΔZ) en fonction des valeurs d'impédance de fin d'expiration (EELI) pour au moins un poumon afin d'obtenir une fonction ajustée (ΔZ_{(idéal)} (EELI)), en particulier une fonction linéaire ΔZ_{idéal}= α EELI + β, pour au moins un poumon (2) et déterminer des données représentatives d'une distribution des valeurs d'amplitude d'impédance (ΔZ) par rapport aux valeurs ajustées (ΔZ_{idéal)}.

11. Procédé selon la revendication 10, comprenant l'étape de prédétermination d'un critère sur la base d'un groupe contrôle de patients, et de préférence l'enregistrement du critère dans une mémoire de données.

12. Procédé selon la revendication 10 ou 11, comprenant l'étape consistant à déterminer, pour chaque pixel, une valeur de déviation (Strain_{EIT}) représentant l'écart de la valeur d'amplitude d'impédance (ΔZ) par rapport à la valeur ajustée (ΔZ_{idéal}), en particulier le rapport (ΔZ/ΔZ_{idéal}) entre les valeurs d'amplitude d'impédance (ΔZ) et la valeur ajustée (ΔZ_{idéal}) à la même valeur d'impédance de fin d'expiration (EELI).

13. Procédé selon la revendication 12, comprenant l'étape consistant à déterminer une quantité de pixels (1) pour lesquels la valeur de déviation (Strain_{EIT}) est supérieure à un seuil prédéterminé et/ou pour lesquels la valeur de déviation (Strain_{EIT}) se situe en dehors d'une plage prédéterminée.

14. Procédé selon l'une des revendications 10 à 13, comprenant l'étape consistant à fournir ou émettre un signal de sortie pour afficher une représentation des données, en particulier afficher le signal de sortie.

15. Procédé selon l'une des revendications 10 à 14, comprenant l'étape de détermination des données pour chacun des poumons (2) et/ou comprenant l'étape de détermination des données de référence.

16. Produit de programme informatique directement chargeable dans un ordinateur et/ou destinée à être exécuté sur un ordinateur, en particulier dans une unité de calcul et de contrôle d'un dispositif selon les revendications 1 à 9 et/ou un dispositif EIT, dans lequel le produit de programme informatique comprend des portions de code logiciel pour exécuter les étapes d'une méthode selon l'une des revendications 10 à 15.
